(19) Europäisches Patentamt
European Patent Office
Office européen des brevets

(11) Veröffentlichungsnummer: **0 073 480**
**B1**

(12) # EUROPÄISCHE PATENTSCHRIFT

(45) Veröffentlichungstag der Patentschrift: **19.06.85**

(51) Int. Cl.⁴: **C 07 C 143/828**

(21) Anmeldenummer: **82107845.8**

(22) Anmeldetag: **26.08.82**

(54) **Verfahren zur Herstellung von teilweise neuen 1,2-Dihalogenalkyl- und -cycloalkylsulfonylisocyanaten.**

(30) Priorität: **01.09.81 DE 3134548**

(43) Veröffentlichungstag der Anmeldung: **09.03.83 Patentblatt 83/10**

(45) Bekanntmachung des Hinweises auf die Patenterteilung: **19.06.85 Patentblatt 85/25**

(84) Benannte Vertragsstaaten: **BE CH DE FR GB IT LI NL**

(56) Entgegenhaltungen:
**DE - A - 2 126 838**
**DE - A - 2 153 794**
**DE - B - 1 230 016**

(73) Patentinhaber: **HOECHST AKTIENGESELLSCHAFT, Postfach 80 03 20, D-6230 Frankfurt am Main 80 (DE)**

(72) Erfinder: **Willms, Lothar, Dr., Grüner Weg 4, D-5463 Unkel (DE)**
Erfinder: **Günther, Dieter, Dr., Nachtigallenweg 1a, D-6233 Kelkheim (Taunus) (DE)**
Erfinder: **Hüttelmaier, Thomas, Dr., Kuckucksweg 19, D-6233 Kelkheim (Taunus) (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 073 480 B1

BUNDESDRUCKEREI BERLIN

**Beschreibung**

Die Erfindung betrifft ein Verfahren zur Herstellung von teilweise neuen 1,2-Dihalogenalkyl- und -cyloalkylsulfonylisocyanaten und deren Verwendung zur Herstellung von Pflanzenschutzmitteln.

Einige Dihalogenalkyl- und -cycloalkylsulfonylisocyanate sind bereits aus dem Stand der Technik bekannt. Gemäß den Angaben von Günther et al. in »Radikalinduzierte Reaktionen von Olefinen mit Chlorsulfonylisocyanat«, Chem. Ber. 103, 663 bis 669 (1970) oder in den DE-B-1 211 165 und 1 226 565 kann man Chlorsulfonylisocyanat an ungesättigte Kohlenwasserstoffe anlagern, wobei man in Gegenwart von Radikalbildnern je nach Reaktionsführung 2-Chloralkyl- bzw. -cycloalkylsulfonylisocyanate oder 3-Oxo-2-[2-chloralkyl]-isothiazolidin-1,1-dioxide erhält.

Die Herstellung von 1-Halogen-2-chloralkyl- bzw. -cycloalkylsulfonylisocyanaten ist nach dem beschriebenen Verfahren nicht möglich. Vielmehr entstehen bei der radikalischen Addition von Chlorsulfonylisocyanat an Vinylchlorid 2,2-Dichlorethylsulfonylisocyanat, 2,4,4-Trichlorbutylsulfonylisocyanat und 2,4,6,6-Tetrachlorhexylsulfonylisocyanat, nicht jedoch 1,2-Dichlorethylsulfonylisocyanat (siehe DE-B-1 226 565).

Auch einige 1,2-Dihalogenalkylsulfonylisocyanate sind bereits bekannt. Die Perfluoralkylsulfonylisocyanate gemäß der DE-A-2 153 794 sollen u. a. auch als pestizid wirksame Verbindungen eingesetzt werden können. Bei der Herstellung von Benzimidazolen nach der DE-A-2 126 838 werden als Ausgangskomponenten auch 1,2-Dichloralkylsulfonylisocyanate (Dichlorethylsulfonylisocyanat) eingesetzt bzw. als geeignet für die Synthese bezeichnet (Dichlorpropyl- und Trichlorethylsulfonylisocyanat). Die Herstellung der Fluorverbindung geht von Perfluoralkylsulfonamid aus und wegen der Herstellung der Dichlorderivate wird auf die vorstehend zitierten Veröffentlichungen verwiesen.

Aufgabe der vorliegenden Erfindung ist es, ein Verfahren zu entwickeln, mit dessen Hilfe es gelingt, eine Vielzahl von verschiedensten 1,2-Dihalogenalkyl- oder -cycloalkylsulfonylisocyanaten zu synthetisieren.

Die Erfindung geht aus von dem bekannten Verfahren zur Herstellung von Dihalogenalkyl- und -cycloalkylsulfonylisocyanaten. Das erfindungsgemäße Verfahren ist dann dadurch gekennzeichnet, daß man 1,2- ungesättigte Alkenyl- oder Cycloalkenylsulfonylisocyanate der allgemeinen Formel (I)

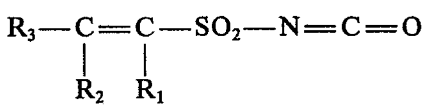
(I)

mit Halogen zu den 1,2-Dihalogenalkyl- oder 1,2-Dihalogencycloalkylsulfonylisocyanaten der allgemeinen Formel (II)

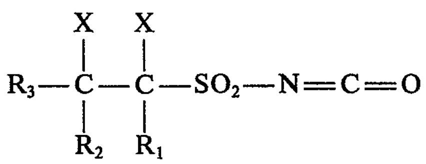
(II)

umsetzt, wobei die Substituenten folgende Bedeutung haben:

$R_1$ und $R_2$ sind unabhängig voneinander H, Halogen, $(C_1-C_4)$-Alkoxy, ein gegebenenfalls durch bis zu 4 Halogen substituierter gesättigter oder ungesättigter, verzweigter oder unverzweigter Alkylrest mit 1 bis 6 C-Atomen, der gegebenenfalls durch ein O-Atom unterbrochen sein kann, oder

$R_1$ und $R_2$ bilden zusammen mit den beiden benachbarten C-Atomen einen 5- bis 7gliedrigen alicyclischen Ring, der gegebenenfalls ungesättigt und/oder durch 1 bis 3 Halogen oder 1 bis 2 $(C_1-C_3)$-Alkylgruppen substituiert ist, wobei $R_1$ und $R_2$ insgesamt bis zu 11 C-Atome enthalten können,

$R_3$ ist H, Halogen oder $(C_1-C_4)$-Alkyl und

X ist Halogen.

Unter den im Verfahren herstellbaren Verbindungen der allgemeinen Formel (II) sind alle die neu, in denen nicht a) X=F oder b) X=Cl und $R_1$, $R_2$, $R_3$=H; $R_1$, $R_2$=H, $R_3$=Cl oder $CH_3$; oder $R_1=CH_3$, $R_2$=H, $R_3$=Cl bedeuten.

Bevorzugt wird das Verfahren zur Herstellung von 1,2-Dichlor- bzw. 1,2-Dibromalkyl- bzw. -cycloalkylsulfonylisocyanaten angewendet.

Bei der Halogenaddition entstehen je nach Art der Substituenten $R_1$, $R_2$ und $R_3$ und der Konfiguration der eingesetzten ungesättigten Verbindungen unter Umständen diastereomere Verbindungen, deren Reindarstellungen z. B. durch fraktionierte Destillation in einigen Fällen möglich ist (vgl. z. B. Beispiel 2).

Beispielsweise lassen sich folgende Verbindungen nach dem erfindungsgemäßen Verfahren herstellen:

1,2-Dibromethylsulfonylisocyanat (Sdp. 74° C/6,7 Pa);
1,2,2-Trichlorethylsulfonylisocyanat (Sdp. 60° C/8 Pa);
threo-1,2-Dibrompropylsulfonylisocyanat (Sdp. 74° C/6, 7 Pa);
1,2,3-Trichlorpropylsulfonylisocyanat;
1,2-Dichlor-3-methoxy-propylsulfonylisocyanat;
1,2-Dichlor-1-methylpropylsulfonylisocyanat (Sdp. 42° C/1,33 Pa);
1,2-Dibrom-1-methylpropylsulfonylisocyanat (Sdp. 62° C/4 Pa);
1,1,2-Trichlorpropylsulfonylisocyanat;
1,2-Dibrom-4,4-dichlorbutylsulfonylisocyanat;
1,2-Dibrompentylsulfonylisocyanat;
1,2-Dibromhexylsulfonylisocyanat;
1,2,3-Trichlor-2-methylpropylsulfonylisocyanat;
1,2-Dichlor-1-chlormethylpropylsulfonylisocyanat;
1,2-Dibrom-cyclohexylsulfonylisocyanat (Sdp. 110° C/133 Pa);
1,2,6-Trichlorcyclohexylsulfonylisocyanat.

Als Ausgangsmaterialien lassen sich nach dem erfindungsgemäßen Verfahren beispielsweise Vinylsulfonylisocyanat, 2-Chlorvinylsulfonylisocyanat, Propenylsulfonylisocyanat, 3-Chlor-propenylsulfonylisocyanat, 1-Methylpropenylsulfonylisocyanat, Butenyl-1-sulfonylisocyanat, 4-Chlorbutadienylsulfonylisocyanat, 4,4-Dichlorbutenyl-1-sulfonylisocyanat oder Cyclohexenyl-1-sulfonylisocyanat einsetzen. Die genannten 1,2-ungesättigten Alkenyl- bzw. Cycloalkylsulfonylisocyanate sind leicht zugänglich und können aus den entsprechenden 2-Chlor-alkyl- bzw. -cycloalkylsulfonylisocyanaten durch Abspaltung von Chlorwasserstoff hergestellt werden DE-B-1 230 016; DE-B-1 568 640).

Das Verfahren läßt sich beispielsweise so durchführen, daß man die 1,2-ungesättigten Alkenyl- bzw. Cycloalkenylsulfonylisocyanate mit dem entsprechenden Halogen unter guter Durchmischung vorteilhaft in Gegenwart von inerten Lösungs- oder Verdünnungsmittel und von radikalischen Startmitteln umsetzt. Als erstere sind beispielsweise Benzol, Toluol, Heptan, Tetrachlorkohlenstoff, Chloroform, Dichlormethan, 1,1,2-Trichlorethan und Gemische geeignet. Als Radikalstartmittel (Katalysatoren) kommen solche Verbindungen in Frage, die in der Lage sind, unter den Reaktionsbedingungen Radikale zu erzeugen, wie z. B. Peroxide oder Azoverbindungen. Genannt seien Azo-bis-isobutyronitril, Diisopropylperoxidicarbonat, Acetylcyclohexansulfonylperoxid, Di-tert.-butyl-peroxid und Benzoylperoxid. Der Katalysator kann in Mengen von 0,01 bis 20 Gewichtsprozent, vornehmlich von 0,01 bis 5 Gewichtsprozent, bezogen auf das eingesetzte Sulfonylisocyanat, angewandt werden. Ferner kann man die Radikale auch durch Bestrahlung der Reaktionsmischung beispielsweise mit UV-Licht erzeugen.

Das Molverhältnis der Reaktionskomponenten ist nicht kritisch und kann bei geeigneter Versuchsführung in weiten Grenzen variiert werden. Wird das 1,2-ungesättigte Alkenyl- bzw. Cycloalkenylsulfonylisocyanat im Überschuß eingesetzt, so kann es bei der Aufarbeitung z. B. durch Destillation zurückgewonnen werden.

Aus ökonomischen Gründen werden jedoch Molverhältnisse (Sulfonylisocyanat : Halogen) von 0,8 : 1 bis 1 : 4, insbesondere 1 : 1 bis 1 : 3 bevorzugt. Weitere gegebenenfalls im aliphatischen bzw. cycloaliphatischen Rest der 1,2-ungesättigten Alkenyl- bzw. Cycloalkenylsulfonylisocyanate vorhandene ungesättigte Gruppierungen werden bei Verwendung eines Überschusses an Halogen je nach Reaktionsführung ebenfalls halogeniert.

Die Reaktionstemperatur kann innerhalb weiter Grenzen variiert werden. Aus praktischen Gründen wählt man eine Temperatur im Bereich von ca. —600° C bis 140° C, wobei der Bereich von —10° bis 80° C bevorzugt ist.

Die Reaktionszeit richtet sich nach den Bedingungen, unter denen das erfindungsgemäße Verfahren durchgeführt wird. In der Regel liegt sie zwischen 1 und 48 Stunden.

Üblicherweise wird die Umsetzung bei Normaldruck durchgeführt. Es kann jedoch auch unter Druck gearbeitet werden, wobei Drücke von 1 bis 1000 at angewendet werden können.

Der Halogenstrom kann gegebenenfalls durch Zumischen von Inertgasen, wie z. B. Stickstoff oder Argon, verdünnt werden.

Die erfindungsgemäßen Verbindungen sind neue, reaktive Substanzen, welche als Zwischenprodukte für Pflanzenschutzmittel, Pharmazeutika und Textilhilfsmittel geeignet und zahlreichen weiteren Umsetzungen zugänglich sind. Zum Beispiel eröffnet ihre Umsetzung mit heterocyclischen Aminen einen neuen, vereinfachten Weg zur Herstellung von Alkyl- und Cycloalkylsulfonylharnstoffen, welche hervorragende herbizide Eigenschaften aufweisen (vgl. die DE-A-3 111 451 und 3 131 489), in hohen Ausbeuten. Überraschenderweise erfolgt bei dieser Umsetzung auch bei Anwendung von überschüssigem Amin kein Austausch des $\alpha$-Halogenatoms, wie dies z. B. von den analogen Halogenalkyl-isocyanaten berichtet wurde [J. org. Chem. 28, 1830 (1963)].

## Beispiel 1

399 g (3 mol) Vinylsulfonylisocyanat werden in 1,2 l Dichlormethan unter Belichtung mit einer UV-Hochdrucktauchlampe mit Chlor begast. Die Innentemperatur wird durch Kühlen zwischen 20° C und 30° C gehalten. Nach 1 Stunden ist die Chloraufnahme (Verbrauch ca. 215 g) beendet und die Reaktionsmischung wird am Rotationsverdampfer vom Lösungsmittel befreit. Der Rückstand wird bei 100° C/26,6 Pascal einer Dünnschichtdestillation unterworfen, wobei man 368 g (1,8 mol; 60% d. Th.) an 1,2-Dichlorethylsulfonylisocyanat erhält. Die erneute Destillation über eine Vigreux-Kolonne liefert ein Produkt vom Sdp. 43° C/7,98 Pascal.

Analyse: 1,2-Dichlorethylsulfonylisocyanat $C_3H_3Cl_2NO_3S$ (MG 204,05)

| | | | | | |
|---|---|---|---|---|---|
| ber.: | C 17,7; | H 1,5; | Cl 34,8; | N 6,9; | S 15,7% |
| gef.: | C 17,8; | H 1,9; | Cl 34,2; | N 7,0; | S 16,0% |

## Beispiel 2

200 g (1,36 mol) Propenylsulfonylisocyanat werden in 0,8 l Dichlormethan gelöst und nach Zusatz von 1 ml Diisopropyl-peroxidicarbonat-Lösung (40%ige Lösung in Phthalat) chloriert. Die Temperatur wird durch Kühlung auf 25° C bis 35° C gehalten. Nach 4 Std. hat das Reaktionsgemisch ca. 120 g Chlor aufgenommen. Das Lösungsmittel wird abdestilliert und der Rückstand bei 60° C/7,98 Pascal einer Dünnschichtdestillation unterworfen. Man erhält 272 g eines Rohproduktes, das sich durch fraktionierte Destillation in zwei Produkte zerlegen läßt:

164 g threo-1,2-Dichlorpropylsulfonylisocyanat,
Sdp. 42—43° C/1,33 Pascal (0,75 mol, 56% d. Th.);
80 g erythro-1,2-Dichlorpropylsulfonylisocyanat,
Sdp. 55° C/1,33 Pascal (0,37 mol, 27,2% d. Th.);

Analyse: threo-1,2-Dichlorpropylsulfonylisocyanat $C_4H_5Cl_2NO_3S$ (MG 218,07)

| | | | | | |
|---|---|---|---|---|---|
| ber.: | C 22,0; | H 2,3; | Cl 32,5; | N 6,4; | S 14,7% |
| gef.: | C 22,4; | H 2,3; | Cl 31,5; | N 6,6; | S 14,7% |

erythro-1,2-Dichlorpropylsulfonylisocyanat $C_4H_5Cl_2NO_3S$ (MG 218,07)

| | | | |
|---|---|---|---|
| gef.: | C 22,4; | H 2,3; | Cl 32,3%. |

## Beispiel 3

38,6 g (0,2 mol) 4-Chlorbutadienylsulfonylisocyanat werden in 100 ml Chloroform gelöst und bei Raumtemperatur in 1 Std. mit 64 g (0,4 mol) Brom in 100 ml Chloroform versetzt. Man rührt zunächst 1 Std. bei Raumtemperatur und anschließend 9 Std. bei 60° C nach. Nach Abdestillieren des Lösungsmittels erhält man 68,9 g (67% d. Th.) an 1,2,3,4-Tetrabrom-4-chlorbutylsulfonylisocyanat in Form eines gelblichen Öles, welches nicht ohne Zersetzung destillierbar ist (Brechungsindex $n_D^{20}$: 1,5698).

## Beispiel 4

100 g (0,53 mol) Cyclohexenyl-1-sulfonylisocyanat werden in Analogie zu Beispiel 1 in Dichlormethan bei einer Temperatur von —10° C bis 0° C chloriert. Nach destillativer Aufarbeitung erhält man 54,6 g (0,21 mol, 40% d. Th.) 1,2-Dichlorcyclohexylsulfonylisocyanat vom Sdp. 100° C/6,65 Pascal

Analyse: 1,2-Dichlorcyclohexylsulfonylisocyanat $C_7H_9Cl_2NO_3S$ (MG 258,12)

| | | | | |
|---|---|---|---|---|
| ber.: | C 32,6; | H 3,5; | Cl 27,5; | S 12,4% |
| gef.: | C 32,4; | H 3,6; | Cl 26,5; | S 12,5%. |

**Patentansprüche**

1. Verfahren zur Herstellung von Dihalogenalkyl- und -cycloalkylsulfonylisocyanaten, dadurch gekennzeichnet, daß man 1,2-ungesätigte Alkenyl- oder Cycloalkenylsulfonylisocyanate der allgemeinen Formel (I)

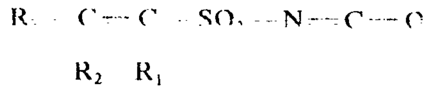

(I)

mit Halogen zu den 1,2-Dihalogenalkyl- oder 1,2-Dihalogencycloalkylsulfonylisocyanaten der allgemeinen Formel (II)

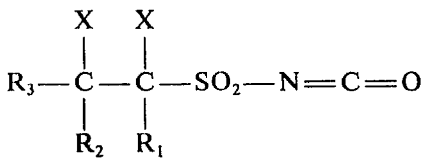

(II)

umsetzt, wobei die Substituenten folgende Bedeutung haben:

$R_1$ und $R_2$ sind unabhängig voneinander H, Halogen, $(C_1-C_4)$-Alkoxy, ein gegebenenfalls durch bis zu 4 Halogen substituierter gesättigter oder ungesättigter, verzweigter oder unverzweigter Alkylrest mit 1 bis 6 C-Atomen, der gegebenenfalls durch ein O-Atom unterbrochen sein kann, oder

$R_1$ und $R_2$ bilden zusammen mit den beiden benachbarten C-Atomen einen 5- bis 7gliedrigen alicyclischen Ring, der gegebenenfalls ungesättigte und/oder durch 1 bis 3 Halogen oder 1 bis 2 $(C_1-C_3)$-Alkylgruppen substituiert ist, wobei $R_1$ und $R_2$ insgesamt bis zu 11 C-Atome enthalten können,

$R_3$ ist H, Halogen oder $(C_1-C_4)$-Alkyl und

X ist Halogen.

2. Verfahren nach Anspruch 1, dadurch gekennzeichnet, daß X = Cl oder Br ist und die 1,2-Dichlor- und 1,2-Dibromalkyl- oder -cycloalkylsulfonylisocyanate hergestellt werden.

3. Verfahren nach Anspruch 1 oder 2, dadurch gekennzeichnet, daß $R_1$ und $R_2$ unabhängig voneinander H, Cl, Br, $(C_1-C_4)$-Alkyl, $(C_1-C_2)$-Halogenalkyl, Methoxymethyl oder zusammen einen Tri- oder Tetramethylenrest bedeuten.

4. 1,2-Dihalogenalkyl- und 1,2-Dihalogencycloalkylsulfonylisocyanate der allgemeinen Formel (II)

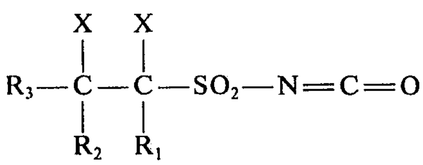

(II)

mit der Bedeutung der Substituenten gemäß Anspruch 1, ausgenommen sind Verbindungen, in denen a) X = F oder b) X = Cl und $R_1$, $R_2$, $R_3$ = H; $R_1$, $R_2$ = H, $R_3$ = Cl oder $CH_3$; oder $R_1$ = $CH_3$, $R_2$ = H, $R_3$ = Cl bedeuten.

5. Verwendung der Verbindungen gemäß Anspruch 4 zur Herstellung von Pflanzenschutzmitteln.

## Claims

1. A process for the preparation of dihalogenoalkyl- and cycloalkylsulfonylisocyanates, which comprises reacting 1,2-unsaturated alkenyl- or cycloalkenylsulfonylisocyanates of the general formula (I)

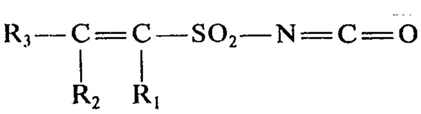

(I)

with halogen by obtaining 1,2-dihalogenoalkyl- or 1,2-dihalogenocycloalkylsulfonylisocyanates of the general formula (II)

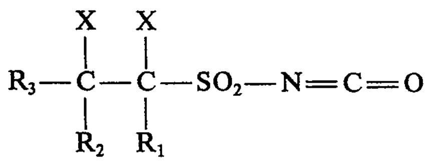

(II)

whereby the substituents have the following meanings:

$R_1$ and $R_2$, independent of each other, are hydrogen, halogen, $(C_1-C_4)$alkoxy, saturated or unsaturated, branched or linear $C_1-C_6$alkyl optionally substituted by up to 4 halogen atoms and optionally interrupted by an oxygen atom or $R_1$ and $R_2$ together with both adjacent carbon atoms may form a 5- to 7-membered alicyclic ring optionally unsaturated and/or substituted by 1 to 3 halogen atoms or by 1 to 2 $(C_1-C_3)$alkyl groups,
$R_1$ and $R_2$ having optionally together up to 11 carbon atoms,
$R_3$ is hydrogen, halogen or $(C_1-C_4)$alkyl a and
X is halogen.

2. A process according to claim 1, wherein X=Cl or Br and wherein 1,2-dichloro- and 1,2 dibromoalkylor — cycloalkylsulfonylisocyanates are formed.

3. A process according to claims 1 or 2, wherein $R_1$ and $R_2$ independently of each other denote H, Cl, Br, $(C_1-C_4)$alkyl, $(C_1-C_2)$-halogenoalkyl, methoxymethyl or denote together a tri- or tetramethylene radical.

4. 1,2-Dihalogenoalkyl- and 1,2-dihalogenoalkylsulfonylisocyanates of the general formula (II)

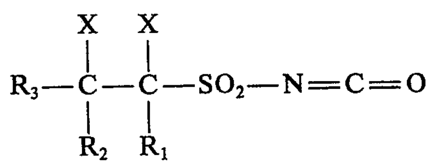

(II)

with the meanings of the substituents of claim 1 with the exception of those compounds wherein a) X=F or b) X=Cl and $R_1$, $R_2$, $R_3$=H; $R_1$, $R_2$=H, $R_3$=Cl or $CH_3$; or $R_1=CH_3$, $R_2$=H, $R_3$=Cl.

5. Use of the compounds according to claim 4 for the preparation of plant protection agents.

**Revendications**

1. Procédé de préparation d'isocyanates de dihalogénoalkylsulfonyles et d'isocyanates de dihalogénocycloalkylsulfonyles, procédé caractérisé en ce qu'on fait réagir des isocyanates d'alcényl-sulfonyles ou de cycloalcényl-sulfonyles insaturés en 1,2 et répondant à la formule générale I:

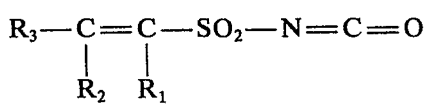

(I)

avec un halogène, de manière à obtenir les isocyanates de dihalogéno-1,2 alkylsulfonyles ou les isocyanates de dihalogéno-1,2 cycloalkylsulfonyles répondant à la formule générale II:

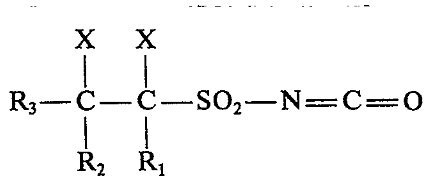

(II)

formules dans lesquelles les divers symboles ont les significations suivantes:

$R_1$ et $R_2$ représentent chacun, indépendemment l'un de l'autre, l'hydrogène, un halogène, un alcoxy en $C_1-C_4$, ou un alkyle en $C_1-C_6$ ramifié ou non ramifié, saturé ou non saturé, éventuellement porteur d'atomes d'halogène en un nombre d'au plus quatre et éventuellement interrompu par un atome $-O-$, ou encore,
$R_1$ et $R_2$ forment ensemble, et avec les deux atomes de carbone voisins, un radical alicyclique qui

contient de 5 à 7 maillons, qui est éventuellement insaturé et/ou qui porte éventuellement de 1 à 3 atomes d'halogène ou 1 ou 2 radicaux alkyles en $C_1-C_3$, $R_1$ et $R_2$ pris ensemble pouvant contenir jusqu'à 11 atomes de carbone,

$R_3$ représente l'hydrogène, un halogène ou un alkyle en $C_1-C_4$, et

X représente un halogène.

2. Procédé selon la revendication 1 caractérisé en ce que X représente un atome de chlore ou de brome et en ce qu'on prépare des isocyanates de dichloro-1,2 alkylsulfonyles, des isocyanates de dichloro-1,2 cycloalkylsulfonyles, des isocyanates de dibromo-1,2 alkylsulfonyles ou des isocyanates de dibromo-1,2 cycloalkylsulfonyles.

3. Procédé selon l'une des revendications 1 et 2, caractérisé en ce que $R_1$ et $R_2$ , représentent chacun, indépendamment l'un de l'autre, H, Cl, Br, un alkyle en $C_1-C_4$, und halogénoalkyle en $C_1$ ou $C_2$ ou un méthoxyméthyle, ou forment ensemble un radical triméthylène ou tétraméthylène.

4. Isocyanates de dihalogéno-1,2 alkylsulfonyles et isocyanates de dihalogéno-1,2 cycloalkylsulfonyles qui répondent à la formule générale II:

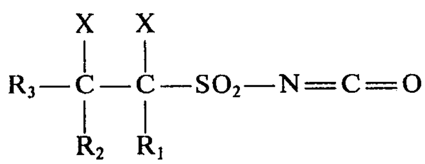

(II)

dans laquelle les divers symboles ont les significations données à la revendication 1, à l'exception des composés dans lesquels a) X=F ou b) X=Cl et $R_1$, $R_2$, $R_3$=H; $R_1$, $R_2$=H; $R_3$=Cl ou $CH_3$; ou $R_1$=$CH_3$, $R_2$=H, $R_3$=Cl.

5. Application des composés selon la revendication 4 à la préparation de produits phytosanitaires.